Europäisches Patentamt

European Patent Office

Office européen des brevets

(19)

(11) Publication number: **0 060 681**
**B1**

(12) **EUROPEAN PATENT SPECIFICATION**

(45) Date of publication of patent specification: **21.01.87**

(51) Int. Cl.⁴: **G 01 N 25/28, G 01 N 33/00**

(21) Application number: **82301229.9**

(22) Date of filing: **11.03.82**

(54) A combustible gas analyzer.

(30) Priority: **17.03.81 US 244537**
**17.03.81 US 244538**
**17.03.81 US 244539**

(43) Date of publication of application:
**22.09.82 Bulletin 82/38**

(45) Publication of the grant of the patent:
**21.01.87 Bulletin 87/04**

(84) Designated Contracting States:
**DE FR GB IT NL**

(56) References cited:
**EP-A-0 008 151**
**DE-B-1 296 838**
**DE-B-2 400 246**
**GB-A-1 555 224**
**US-A-4 134 289**

The file contains technical information
submitted after the application was filed and
not included in this specification

(73) Proprietor: **HONEYWELL INC.**
**Honeywell Plaza**
**Minneapolis Minnesota 55408 (US)**

(72) Inventor: **Szonntagh, Eugene L.**
**6327 Fairfield Drive**
**Flourtown Pennsylvania 19031 (US)**
Inventor: **Pearman, A. Noel J.**
**2087 Bayard Avenue**
**St. Paul Minnesota 55116 (US)**
Inventor: **Kude, William B.**
**10225 - 39th Avenue North**
**Plymouth Minnesota 55441 (US)**

(74) Representative: **Frohwitter, Bernhard, Dipl.-Ing.**
**Bardehle-Pagenberg-Dost-Altenburg & Partner**
**Patent- und Rechtsanwälte Galileiplatz 1**
**Postfach 86 06 20**
**8000 München 80 (DE)**

## Description

The present invention is directed to a combustible gas analyzer and more specifically, to a gas analyzer for determining the heating Wobbe Index of combustible gases and a method for analysing such a gas.

The well-known Wobbe Index of a combustible gas is defined as the amount of heat released by a burner of constant orifice, McGraw-Hill Dictionary of Science, 1979 and stated mathematically by the following relation:

$W = H/\sqrt{SG}$ where H is the caloric combustion value of the gas per unit volume, e.g. $kJ/m^3$, and SG is specific gravity of the combustible gas relative to air. The Wobbe Index is a quantity used in heating technology since different combinations of gases supplied to a gas heated apparatus under the same pressure will provide equal heat production from the apparatus, whereby the apparatus does not need to be readjusted, as long as the Wobbe Index is maintained at a predetermined value. For example, if a mixture of gases from different sources is burned in an industrial heating operation, the gases must be mixed in such proportion that a gas is obtained having a constant Wobbe Index.

One method of determining the Wobbe Index of the gas involves the combination of calorimeter, a density meter and a computing circuit, e.g., a microprocessor. These parts, while they may be combined into a single instrument, produce an overall device which is very costly and exhibits a sluggish operation whereby rapid changes in the gas mixture are measured at a slow rate and the resulting correction, if necessary, is also applied at a corresponding slow rate.

It has been discovered that when gas mixtures having different compositions and different Wobbe indices are burned with equal quantities of air, the oxygen content of the exhaust gas show a direct correlation with the Wobbe Index. Accordingly, for purposes of measurement and control, it is not necessary to measure the Wobbe Index as such and it is sufficient to measure only the oxygen content in the exhaust gas. One apparatus for producing this type of operation is known from EP—A1—8151, and includes a sampling line containing a flow control nozzle for withdrawing a gas sample, a means for adjusting the gas sample so that the pressure difference through this control nozzle has an adjustable constant value, a means for feeding a constant volume of air into the gas stream sample, a combustion chamber, a burner in the combustion chamber to completely burn the gas-air mixture, an outlet for the burned gas from the combustion chamber and an oxygen sensor in the combustion chamber to sense the oxygen content of the exhaust gas. This oxygen content is a quantity which is correlated to the Wobbe Index of the measured gas. However, by actually measuring or providing a quantity which represents the Wobbe Index the thermal delivery rate, i.e. kJ/min, could be obtained to control the industrial heating operation. Thus, the measuring apparatus providing such a Wobbe Index measurement would provide an efficient means of "feed forward" control to a furnace requiring a constant BTU per minute input without regard to changes in gas supply composition, density and kJ content.

The means for producing the required adjustable but constant flow rates (i.e. means for producing a flow rate which is adjustable but is constant for any given setting of the adjustment) is known from for example GB—A—1 555 224 (where that means is used to control a common supply to a pair of feed pipes, to which variable amounts of air are fed, and which feed a pair of burners, the system including feedback means to keep the temperature different between the two flames constant).

The cited specification EP—A1—8151 also describes a fuel control system in which the Wobbe Index of a mixture of fuels is measured, and the ratio of different fuels is varied accordingly so as to maintain the Wobbe Index constant.

The known Wobbe Index measuring system of EP—A1—8151 preferably requires a cheap and efficient oxygen sensor, and a convenient form of oxygen sensor is the zirconium oxide type (known from, for example, DE—B—2 400 246). However, if this type of sensor is used, we have found that its output is decidedly temperature sensitive. The known system therefore suffers from the drawback that the temperature of the combustion gases has to be controlled to a constant value (or the temperature has to be sensed and used to correct the oxygen sensor output).

We have discovered that if the flow rate of the fuel is controlled to as to achieve substantially stoichoimetric combustion, the temperature sensitivity of a zirconium oxide type sensor is substantially reduced. Furthermore, we have discovered that under such conditions, particular forms of such sensors gives still further reduced temperature sensitivity.

Accordingly the invention provides a combustible gas analyzer for determining the Wobbe Index of said gas comprising: means for supplying air and combustible gas at controlled flow rates; means for mixing the air and combustible gas; combustion means for burning the mixture; and a detector for detecting the oxygen content of the combustion products, characterized by: a valve for controlling the flow of combustible gas to the mixing means; control means responsive to the detector to control the valve to achieve an oxygen content of the combustion products corresponding to substantially stoichiometric combustion; and a monitor for monitoring the gas flow restricting effect of the valve to enable the detection or indication of the Wobbe Index of said combustible gas.

According to another aspect of the invention a method for analyzing a combustible gas to determine the Wobbe Index of the gas is provided as disclosed in claim 7.

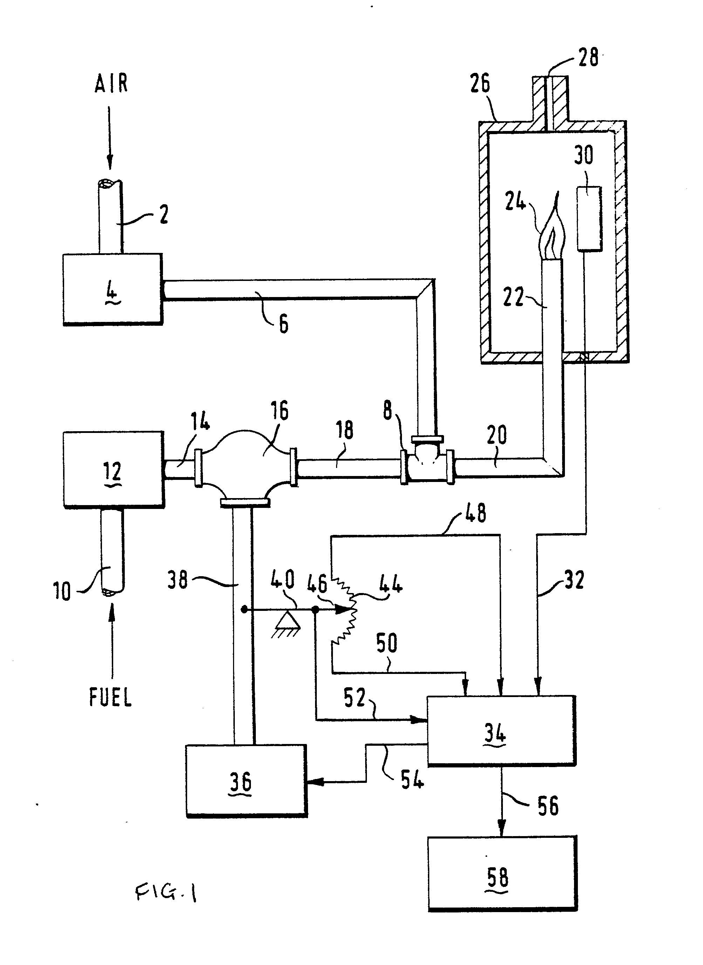

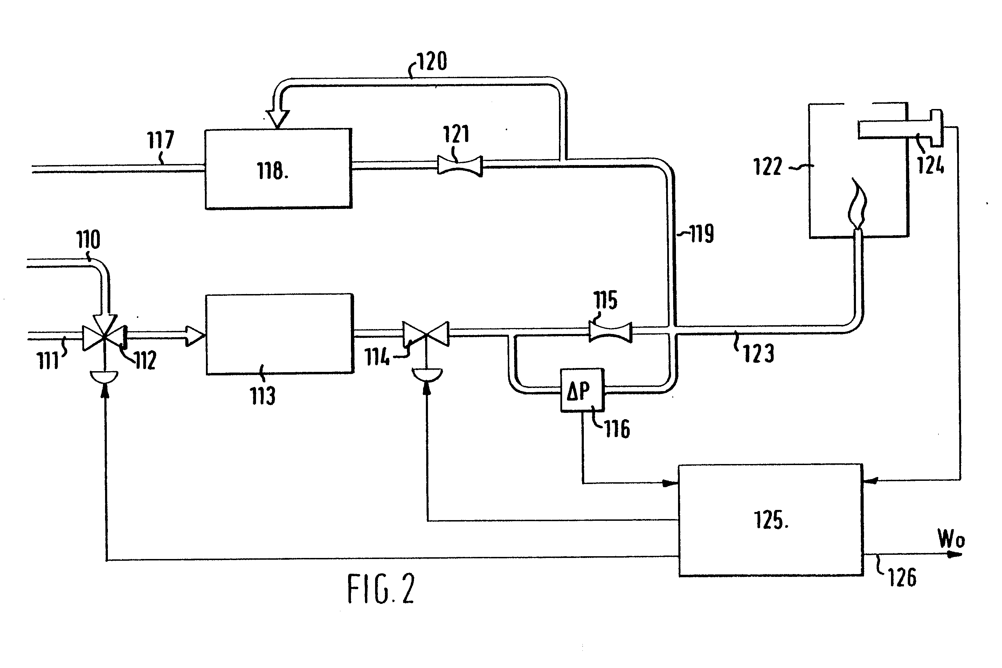

An embodiment of the present invention will now be described in connection with the accompanying drawings, in which the Figures 1 and 2 are pictorial illustrations of two combustible gas analyzers according to the present invention for measuring the Wobbe Index of the gas.

Referring to the Figure 1, the gas analyzer is supplied with air and combustible gas through pipelines 2 and 10. The pipeline 2 is connected to the input of a first conventional pressure regulator 4 while the output of the pressure regulator 4 is connected to an output pipeline 6. The output pipeline 6 is connected to one input of a mixing valve 8. In a conventional fashion, the pipeline 6 is arranged to contain a fixed orifice or restriction (not shown) which is the dominating downstream restriction in the flowline supplied by the output of the pressure regulator 4. Such a fixed orifice provides a means for achieving a steady flow rate of the air from the pressure regulator 4 to form a steady flow rate air supply. Concurrently, a supply (not shown) of a combustible gas is arranged to supply a fuel gas through the second input pipeline 10 connected to the input of a second conventional pressure regulator 12. The output of the second pressure regulator 12 is supplied through an output pipeline 14 to a control valve 16. As in the case of the previously mentioned orifice in the pipeline 6, the valve 16 is arranged to provide an orifice which, while variable, is the dominating downstream restriction in the flowline supplied by the output of the second pressure regulator 12. Since a variation in the flow rate of the fuel gas is desired, a different steady flow rate is achieved for each stable setting of the valve 16 in a similar way to that provided by the orifice in the pipeline 6. The output of the control valve 16 is supplied through an output pipeline 18 to a second input of the mixing valve.

An output of the mixing valve 8 is supplied through pipeline 20 to a burner jet 22 for producing a combustion flame 24. The burner jet 22 and the combustion flame 24 are located within a housing 26 providing a combustion chamber for the combustion flame 24. Also located within the housing 26 would be means for initiating the burning of the combustible gas by means of a spark or other device and means for detecting the presence of the flame 24 to provide a control for the flame initiating device. Such devices are well-known and accordingly have not been shown. The exhaust gases from the combustion chamber 26 are allowed to escape through a restricted opening 28 in the wall of the combustion chamber 26. The aforesaid restrictions provided by the fixed restriction in the pipeline 6 and by the variable restriction of the valve 16 are each effective to provide a greater restrictive effect than that imposed by the other flowline elements, as previously mentioned, including the flow impediments imposed by the mixing connector 8, the burner 22 and the exhaust port 28. A well-known zirconium oxide detector 30 is located adjacent to the flame 24 to effect a detection of the oxygen content of the end products of the combustion process to determine a preselected combustion state, e.g. stoichiometric combustion. The output of the zirconium oxide detector 30 is applied over an output line 32 to an input of a data processing system 34 which may incorporate a microprocessor.

The valve 16 is driven by a valve motor 36 connected thereto through a valve stem 38. The valve stem 38 is connected to an apparatus for providing an indication of the position of the valve stem 38. An example of an apparatus for providing such an indication includes a lever arm 40 attached to the valve stem 38 and arranged to drive the slider 46 of a potentiometer across the resistance element 44 of a potentiometer. The resistance element 44 of the potentiometer is connected by electrical lines 48 and 50 to the data processor 34 while the slider 46 is connected by an electrical line 52 to the data processor 34. The data processor 34 is arranged to use the input signal from the zirconium oxide detector 30 to produce a control signal on output line 54 for controlling the operation of the valve motor 36. Concurrently, the data processor 34 may be used to produce an output signal in response to a signal from the potentiometer 42 on a data output line 56 which is connected to a recorder display 58 for displaying the position of the valve stem 38 as an indication of the Wobbe Index of the fuel gas supplied over the fuel inlet line 10.

In operation, the air supply controlled through the first pressure regulator 4 is supplied to the mixing valve 8 at a constant flow rate in combination with the fuel gas supplied through the second pressure regulator 12 and the control valve 16. The combustion of the fuel gas in the presence of the air is effected by the flame 24 in the combustion chamber 26. The oxygen content of the combustion products or gases is detected by the zirconium oxide detector 30. Since the control valve 16 functions as a variable orifice, the output signal supplied to the recorder display 58 on output line 56 which is representative of the position of the valve motor 36 is a measurement of the Wobbe Index of the fuel gas. This is the result of having a short orifice which produces an effect dependent on the specific gravity of the fluid flowing therethrough. In the combustible gas measuring arrangement described above, the variable orifice produced by the valve 16 is the only significant restriction prior to the burner 22 in the flowline containing the valve 16. In such an arrangement, the calorific value, e.g., kJ of the gas being burned at the burner 22 will be altered if fuels of different specific gravity are introduced from the fuel line 10. In other words since:

$$H = W/\sqrt{SG}$$

then:

$$W = H/\sqrt{SG}$$

According, the measurement of the movement of the valve stem 38 is dependent on the square root of the specific gravity of the fuel relative to air and the calorific content and is, therefore, a direct indication of the Wobbe Index. The value 16 is operated by the data processor 34 by means of the valve motor 36 and valve stem 38 to produce a desired combustion state which is at or near stoichiometric combustion at the flame 24, as discussed more fully later. It should be noted that this desired combustion state is only one point on the combustion curve, and only a repeatability is necessary by returning to the same point for each measurement. The combustion level is detected by the sensor 30 as a result of the minimal amount of oxygen remaining in the combustion products from the flame 24. The representation of the Wobbe Index, i.e., valve position, may be displayed on a suitable display or recorded as a record of the Wobbe Index of the fuel gas since the recorder display 58 may include a hard copy recorder as well as a display apparatus, e.g., cathode ray tube (CRT).

In a modification (not shown), the pressure regulators 4 and 12 are each provided with a feed back line for supplying a pressure feedback signal from T connectors in lines 8 and 18 respectively back to the regulators for controlling the pressure output thereof. By providing feedback pressure from the output of the valve 16, the analyzer/ operator is made independent of specific gravity. Thus, any change in specific gravity would readjust the pressure equilibrium of the regulator 12 to compensate for a specific gravity whereby the valve stem position is a measure of the Wobbe Index. It should be noted that, since the accuracy of the Wobbe Index measurement is dependent on specific gravity of the fuel gas, the proportion of non-combustible gases therein, e.g., helium will effect the accuracy of the measurement. Specifically, the relationship is an inversely proportional one, i.e., the greater the quantity of non-combustible elements, the lower will be the measurement accuracy. However, in a real life application of the present analyzer, e.g., gas user measurements, the non-combustible content is very small which provide a high measurement accuracy of the Wobbe Index, e.g., 1%.

In a second embodiment of the invention, shown in Figure 2, the analyzer includes a sample fuel inlet 110 which, with calibration gas inlet 111, is adapted to supply either sample fuel or calibration gas to the system from suitable sources thereof as through an electrically controlled two-position, three-way valve 112 which admits the selected components to the system through pressure regulator 113. A flow control valve 114, which may be any suitable type of flow control valve which can be accurately modulated electrically, such as a needle valve, is provided to control the sample flow input to the system. A metering orifice 115 in conjunction with a sensitive orifice pressure transducer 116 is utilized to accurately measure the sample fuel input to the system. Combustion air is applied to the system through an air inlet 117, which may be supplied from a combustion air blower or other source of pressurized air. The pressure is regulated by pressure regulator 118 which is backloaded from the common air-gas line 119 as at 120 and a metering orifice 121. The sample burner is supplied with the air-gas mixture through line 123 and the products of combustion are sensed as by sensor 124, preferably of Zirconium Oxide ($ZrO_2$). The necessary control and data processing functions are performed by electronics shown at 125 and the Wobbe Index output, which may be in the form of a digital register, recorder, direct controller or other means is indicated at 126.

It has been found that sensor output is decidely temperature sensitive in the presence of more than minute amounts of oxygen. Thus, when operating in the oxygen rich portion of the $ZrO_2$ electrochemical response curve the temperature of the sensor should also be carefully controlled. When operating on or about the stoichiometric point, however, the output is substantially stable over a wide temperature range, i.e., from about 800°C to 1400°C, and therefore temperature control in the combustion chamber is not critical. In fact, the relative size of the combustion chamber 26 or 122 is normally sized in accord with the amount of fuel to be burned such that the temperature within the chamber is maintained within the limits where the sensor is not temperature sensitive at or about the stoichiometric point of combustion.

The $ZrO_2$ sensor of Figures 1 and 2 can be stabilized with amounts of CaO or $Y_2O_3$ to further stabilize the temperature related response and to enhance the chemical and physical stability of the Zirconium Oxide for use as a sensor. This results in a stable sensor which possesses almost a step-change response at, or about the point of mixture stoichiometry.

As can be seen from the embodiment of Figure 2, the fuel flow rate can be accurately controlled by the motorized needle valve 114 in conjunction with the orifice 115. The air, of course, is also suitably pressure regulated as seen in that figure. After the fuel and air are mixed and burned in the chamber 122, the sensor 124 is used in conjunction with the electronics to achieve and maintain substantially stoichiometric proportions for the combustion of the sample gas by controlling the fuel flow.

When substantially stoichiometric combustion is achieved in the chamber the differential pressure (P) measurement by pressure transducer sensor 116 can be utilized to calculate the actual Wobbe Index (Wo), from the equation

$$Wo = K/\sqrt{P}.$$

**Claims**

1. A combustible gas analyzer for determining the Wobbe Index of said gas comprising means (4, 12, 117, 110) for supplying air and combustible gas at controlled flow rates; means (8, 123) for mixing the air and combustible gas; combustion means (22, 26, 28, 122) for burning the mixture; and a detector (30, 124) for detecting the oxygen content of the combustion products; characterized by: a valve (16, 114) for controlling the flow of combustible gas to the mixing means; control means (34, 125) responsive to the detector to control the valve to achieve an oxygen content of the combustion products corresponding to substantially stoichiometric combustion; and a monitor (40, 44, 46, 50, 115, 116) for monitoring the gas flow restricting effect of the valve to enable the detection or indication of the Wobbe Index of said combustible gas.

2. The analyzer as of Claim 1, characterized in that the valve includes a valve stem (38) arranged to vary a valve orifice by a corresponding movement of the stem and a valve stem drive motor, and wherein the monitor is arranged to produce an output signal representative of the position of the valve stem.

3. The analyzer of Claim 2, characterized by a valve stem movement monitor including a potentiometer (42) having a resistance element (44) and a contact slider (46) in contact with the resistance element and connected to the valve stem to be driven across said resistance element by the valve stem.

4. The analyzer of Claim 1, 2 or 3, characterized in that the means (4, 12) for supplying air and combustible gas at controlled flow rates are pressure regulators each with pressure feedback.

5. The analyzer of Claim 1, characterized in that the monitor includes a metering orifice (115), and a transducer (116) measuring the pressure differential across the orifice.

6. The analyzer of any one of the preceding Claims, characterized in that the detector (30, 124) is of Zirconium Oxide ($ZrO_2$) stabilized by CaO, MgO or $Y_2O_3$.

7. A method for analyzing a combustible gas to determine the Wobbe Index of the gas including the steps of supplying air and combustible gas at controlled flow rates, mixing the air and the gas, burning the mixed gas and air, and detecting the oxygen content of the combustion products resulting from the burning of the gas and air, characterized by controlling the flow rate of the gas by means of a variable valve to achieve substantially stoichiometric combustion and monitoring the size of the restriction imposed by the valve as an indication of the Wobbe Index of the gas to be analyzed.

8. The method of Claim 7 or 8, characterized in that the air and/or gas are each controlled by a pressure regulator operating with pressure feedback.

**Patentansprüche**

1. Analysator zum Bestimmen des Wobbe-Index' brennbaren Gases mit

Mitteln (4, 12; 117, 110) zur Zufuhr von Luft und brennbarem Gas mit geregelter Durchflußmenge;

einer Mischvorrichtung (8; 123) für die Luft und das Brenngas;

einer Verbrennungseinrichtung (22, 26, 28; 122) zum Verbrennen der Mischung; und

einem Detektor (30; 124) für den Sauerstoffgehalt der Verbrennungsprodukte, gekennzeichnet durch

ein Ventil (16; 114) zum Steuern der Zufuhr brennbaren Gases zur Mischvorrichtung;

eine vom Detektor beeinflußte Regeleinrichtung (34; 125) welche das Ventil derart steuert, daß der Sauerstoffgehalt der Verbrennungsprodukte einer im wesentlichen stoichiometrischen Verbrennung entspricht;

und eine Fühlereinrichtung (40, 44, 46, 50; 115, 116) zum Überwachen der Drosselwirkung des Ventils auf den Gasstrom, um den Wobbe-Index des Brenngases festzustellen oder anzuzeigen.

2. Analysator nach Anspruch 1, dadurch gekennzeichnet, daß das Ventil eine Ventilstange (38) zum Verändern des Ventildurchlasses beim Bewegen der Stange sowie einen Ventilstangenantriebsmotor aufweist und daß die Fühlereinrichtung ein der Stellung der Ventilstange entsprechendes Ausgangssignal erzeugt.

3. Analysator nach Anspruch 2, dadurch gekennzeichnet, daß die Fühlereinrichtung für die Ventilstangenbewegung ein Potentiometer (42) mit einem Widerstandselement (44) und einem hierauf liegenden Kontaktschleifer (46) umfaßt, der mit der Ventilstange in Verbindung steht und durch dies längs des Widerstandselements verstellt wird.

4. Analysator nach Anspruch 1, 2 oder 3, dadurch gekennzeichnet, daß die Mittel zur Zufuhr von Luft und Brenngas mit geregelter Durchflußmenge Druckregler (4, 12) mit Druckrückführung sind.

5. Analysator nach Anspruch 1, dadurch gekennzeichnet, daß die Fühlereinrichtung eine Meßdüse (115) und einen die Druckdifferenz an der Düse messenden Umformer (116) umfaßt.

6. Analysator nach einem der vorangehenden Ansprüche, dadurch gekennzeichnet, daß der Detektor (30; 124) aus durch CaO, MgO oder $Y_2O_3$ stabilisiertem Zirkonoxyd ($ZrO_2$) besteht.

7. Analysenverfahren zum Bestimmen des Wobbe-Index' brennbaren Gases mit den Schritten:

Zufuhr von Luft und Brenngas mit geregelter Durchflußmenge;

Mischen von Luft und Gas;

Verbrennen der Gas/Luftmischung;

Feststellen des Sauerstoffgehalts der bei der Verbrennung der Mischung entstandenen Verbrennungsprodukte,

gekennzeichnet durch die Regelung der Durchflußmenge des Gases mit einem Stellventil derart, daß eine im wesentlichen stoichiometrische Verbrennung erfolgt, und

Überwachen des Grades der durch das Ventil bewirkten Drosselung als Anzeige des Wobbe-Index' des zu analysierenden Gases.

8. Verfahren nach Anspruch 7, dadurch gekennzeichnet, daß die Luft und/oder das Gas durch einen Druckregler mit Druckrückführung geregelt werden.

**Revendications**

1. Analyseur de gaz combustible pour déterminer l'Indice de Wobbe du gaz, comprenant des moyens (4, 12, 117, 110) pour fournir de l'air et un gaz combustible à des débits réglés; un moyen (8, 123) pour mélanger l'air et le gaz combustible; un moyen de combustion (22, 26, 28, 122) pour brûler le mélange; et un détecteur (30, 124) pour détecter la teneur en oxygène des produits de combustion; caractérisé par: une soupape (16, 114) pour régler le débit du gaz combustible jusqu'au moyen mélangeur; un moyen de commande (34, 125) sensible au détecteur pour commander la soupape en vue d'obtenir une teneur en oxygène des produits de combustion correspondant essentiellement à une combustion stoechiométrique; et un contrôleur (40, 44, 46, 50, 115, 116) pour contrôler l'effet de limitation de débit de gaz de la soupape pour permettre la détection ou l'indication de l'Indice de Wobbe du gaz combustible.

2. Analyseur selon la revendication 1, caractérisé en ce que la soupape comprend une tige de soupape (38) agencée pour faire varier un orifice de soupape par un déplacement correspondant de la tige et un moteur d'entraînement de tige de soupape, le contrôleur étant agencé pour produire un signal de sortie représentant la position de la tige de soupape.

3. Analyseur selon la revendication 2, caractérisé par un contrôleur de déplacement de tige de soupape incluant un potentiomètre (42) comportant un élément résistant (44) et un curseur de contact (46) en contact avec l'élément résistant et connecté à la tige de soupape pour être entraîné à travers l'élément résistant par la tige de soupape.

4. Analyseur selon l'une quelconque des revendications 1 à 3, caractérisé en ce que les moyens (4, 12) pour fournir de l'air et du gaz combustible à des débits réglés sont des régulateurs de pression avec chacun une réalimentation ou réaction en pression.

5. Analyseur selon la revendication 1, caractérisé en ce que le contrôleur comprend un orifice de mesure (115), et un transducteur (116) pour mesurer la pression différentielle dans l'orifice.

6. Analyseur selon l'une quelconque des revendications 1 à 5, caractérisé en ce que le détecteur (30, 124) est d'oxyde de zirconium ($ZrO_2$) stabilisé par CaO, MgO, ou $Y_2O_3$.

7. Procédé pour analyser un gaz combustible en vue de déterminer l'Indice de Wobbe du gaz, consistant à fournir de l'air et un gaz combustible à des débits réglés, à mélanger l'air et le gaz, à brûler le gaz et l'air mélangés, et à détecter la teneur en oxygène des produits de combustion résultant de l'opération de combustion du gaz et de l'air, caractérisé en ce qu'il consiste à régler le débit du gaz au moyen d'une soupape variable pour obtenir essentiellement une combustion stoechiométrique et à contrôler la grandeur de la limitation imposée par la soupape comme indication de l'Indice de Wobbe du gaz à analyser.

8. Procédé selon la revendication 7, caractérisé en ce que l'air et/ou le gaz sont réglés chacun par un régulateur de pression fonctionnant avec une réalimentation ou réaction en pression.

FIG. 1

FIG.2

0 060 681